# EUROPÄISCHE PATENTSCHRIFT

(11) **EP 3 012 253 B1**
(45) Veröffentlichungstag und Bekanntmachung des Hinweises auf die Patenterteilung: **17.05.2017**
(21) Anmeldenummer: 14189499.8
(22) Anmeldetag: 20.10.2014
(51) Int. Cl.: C07D 307/12, C08K 5/1535

(54) **Tetrahydrofuran-Derivate**
Tetrahydrofuran derivatives
Dérivés de tétrahydrofurane

(43) Veröffentlichungstag der Anmeldung: 27.04.2016
(73) Patentinhaber: BASF SE, 67056 Ludwigshafen am Rhein (DE)
(72) Erfinder: Haaf-Kleinhubbert, Christina, 69502 Hemsbach (DE); Gatti, Michele, 68163 Mannheim (DE); Habraken, Gijsbrecht, Budd Lake, 07828 (US)
(74) Vertreter: BASF IP Association

(56) Entgegenhaltungen:
- EP-A1- 0 043 448
- DE-A1-102010 044 204
- DE-A1-102010 044 206
- US-A- 3 256 254

## Beschreibung

### Gebiet der Erfindung

Die vorliegende Erfindung Tetrahydrofuran-Derivate spezieller Struktur, die sich zur Beschichtung der Oberflächen fester Substrate, insbesondere von Kunststoffen, eignen.

### Stand der Technik

EP-A-043,448 beschreibt Dimethacrylsäureester des 2,5-Dimethyloltetrahydrofurans. Gemäß der Offenbarung auf Seite 4, Zeilen13-14 sind diese Verbindungen hochviskose Substanzen. Die Verbindungen finden Verwendung als wesentlicher Bestandteil von bei Sauerstoff-Ausschluss erhärtenden Klebstoffen beziehungsweise Dichtungsmitteln.

DE-A-10,2010,044,206 beschreibt ein Verfahren zur Herstellung von strahlungshärtbaren (Meth)acrylaten auf Basis von propoxyliertem Glycerin. Dabei wird zunächst (Meth)acrylsäure mit 3- bis 4-fach propoxyliertem Glycerin umgesetzt, gefolgt von einer Entfernung überschüssiger (Meth)acrylsäure aus dem erhaltenen Reaktionsgemisch mittels wäßriger Extraktion.

DE-A-10,2010,044,20 4 beschreibt ein Verfahren zur Herstellung von strahlungshärtbaren (Meth)acrylaten auf Basis von eth oxyliertem Glycerin. Dabei wird zunächst (Meth)acrylsäure mit 2,9- bis 4-fach ethoxyliertem Glycerin umgesetzt, gefolgt von einer Entfernung überschüssiger (Meth)acrylsäure aus dem erhaltenen Reaktionsgemisch mittels Umsetzung mit mindestens einem aromatischen oder aliphatischen, mindestens bifunktionellen Epoxids. US 3256254 beschreibt u.a. die Verwendung von 2,5-Dimethyloltetrahydrofurandiacrylat als Bestandteil von Einbettmassen.

### Beschreibung der Erfindung

Aufgabe der vorliegenden Erfindung war es, Substanzen zur Verfügung zu stellen, die sich Beschichtung der Oberflächen fester Substrate und insbesondere Kunststoffen ("Plastik") eignen, wobei die Aushärtung der Beschichtung durch Strahlenhärtung, insbesondere mit UV-Licht, erfolgt. Die ausgehärteten Beschichtungen, insbesondere die UV-gehärteten Lacke, sollten dabei hohe Härte aufweisen.

Insbesondere sollten die zu entwickelnden Substanzen zugleich folgende drei Kriterien erfüllen:
(1) Die Viskosität der Substanzen als solche sollte unterhalb von 500 mPas liegen (gemessen mit einem Brookfield-Viskosimeter bei 25 °C, Geschwindigkeitsgefälle von 1000 s-1, gemäß DIN EN ISO 3219/A.3).
(2) Die Pendelhärte der Beschichtungen, die resultieren, wenn die Substanzen auf die Oberflächen fester Substrate aufgebracht und mittels UV-Strahlung gehärtet worden sind, soll höher als 70 sec sein (Pendelhärte nach König, gemessen nach DIN 53157; angegeben wird bei dieser Methode die Pendeldämpfung in Sekunden).
(3) Die Haftung auf Plastik soll 0 bis 2, vorzugsweise 0 oder 1, betragen (gemessen nach der Gitterschnitt-Methode nach DIN EN ISO 2409, wobei die G-Werte nach Schulnoten-System im Bereich von 0 bis 5 liegen; dabei stellt 0 den besten und 5 den schlechtesten Wert dar). Gegenstand der Erfindung sind zunächst Tetrahydrofuran-Derivate der Formel (I) 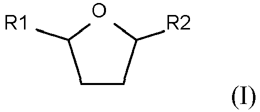 worin der Rest R1 die Bedeutung (CH₂=CH-CO-O-(CHR3-CH₂-O)ₘ-CH₂)- und der Rest R2 die Bedeutung (CH₂=CH-CO-O-(CHR4-CH₂-O)ₙ-CH₂)- hat, wobei die Reste R3 und R4 unabhängig voneinander Wasserstoff oder Methyl bedeuten, und wobei die Maßgabe gilt, dass die Summe der Indices m und n eine Zahl im Bereich von 5 bis 12 ist. Mithin ist eine äquivalente Schreibweise für die Verbindungen (I) die folgenden Struktur (A):

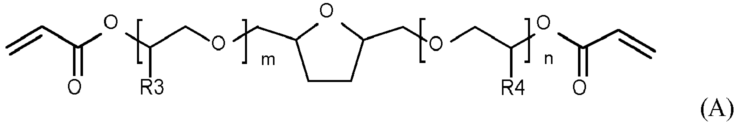

Im Hinblick auf Formel (I) gilt, das die Substituenten R1 und R2 sich entweder auf derselben Seite oder auf verschiedenen Seite der Referenzebene befinden können, die durch den Fünfring gegeben ist. Mit anderen Worten können die Verbindungen (I) in der cis- oder der trans-Form vorliegen. Man würde diese Isomeren entsprechend als cis-2,5-R1-R2-Tetrahydrofuran bzw. als trans-2,5-R1-R2-Tetrahydrofuran bezeichnen.

Die Verbindungen (I) können an sich nach allen dem Chemiker bekannten Verfahren hergestellt werden.

Vorzugsweise stellt man die Verbindungen (I) wie folgt her: Man setzt 2,5-Dimethyloltetrahydrofuran, welchem die Formel (B) zukommt, 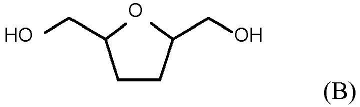 entweder direkt oder nach vorheriger Ethoxylierung und/oder Propoxylierung mit Acrylsäure um. Dabei arbeitet man vorzugsweise mit einem Überschuss an Acrylsäure in einem organischen Lösungsmittel, insbesondere Cyclohexan oder Methylcylohexan, und in Anwesenheit eines sauren Veresterungs-Katalysators, insbesondere Methansulfonsäure, Schwefelsäure oder p-Toluolsulfonsäure.

Vorzugsweise werden technische Gemische des Diols (B) eingesetzt, wobei das (molare) cis/trans-Verhältnis insbesondere im Bereich von 95 : 5 und 5 : 95 liegt. Besonders bevorzugt ist dabei ein (molares) cis/trans-Verhältnis im Bereich von 95: 5 und 50 : 50 und insbesondere im Bereich von 95:5 und 80 : 20.

Nach dem Ende der Veresterung entfernt man eingesetztes Lösungsmittel, vorzugsweise durch Destillation, insbesondere unter vermindertem Druck. Ebenso entfernt man überschüssige Acrylsäure, was auf verschiedene Weise geschehen kann, etwa durch Destillation, Auswaschen oder auf chemischem Wege.

Sofern überschüssige Acrylsäure durch Auswaschen entfernt wird gilt: Für das Auswaschen wird vorzugsweise eine Extraktion mit wässrigem Medium herangezogen (vergleiche hierzu beispielsweise die einschlägige Offenbarung der DE-A-102010044206).

Sofern überschüssige Acrylsäure durch Abfangen auf chemischem Wege entfernt wird gilt: Das Abfangen auf chemischem Wege erfolgt vorzugsweise durch Umsetzung der überschüssigen Acrylsäure mit Epoxidverbindungen, insbesondere mindestens bifunktionellen Epoxidverbindungen (vergleiche hierzu beispielsweise die einschlägige Offenbarung der DE-A-10,2010,044204).
Ein weiterer Erfindungsgegenstand sind Beschichtungsmassen enthaltend ein oder mehrere Tetrahydrofuran-Derivate der Formel (I) 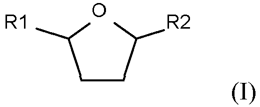 worin der Rest R1 die Bedeutung (CH₂=CH-CO-O-(CHR3-CH₂-O)ₘ-CH₂)- und der Rest R2 die Bedeutung (CH₂=CH-CO-O-(CHR4-CH₂-O)ₙ-CH₂)- hat, wobei die Reste R3 und R4 unabhängig voneinander Wasserstoff oder Methyl bedeuten, und wobei die Maßgabe gilt, dass die Summe der Indices m und n eine Zahl im Bereich von 5 bis 12 ist. Im Hinblick auf die Herstellung der Verbindungen (I) sei auf das oben Ausgeführte verwiesen, sowohl was die allgemeinen Ausführungen angeht, als auch die bevorzugten Ausführungsformen.
Sofern - wie oben ausgeführt - überschüssige Acrylsäure durch Abfangen auf chemischem Wege entfernt wird gilt: Das Abfangen auf chemischem Wege erfolgt vorzugsweise durch Umsetzung der überschüssigen Acrylsäure mit Epoxidverbindungen, insbesondere mindestens bifunktionellen Epoxidverbindungen (vergleiche hierzu beispielsweise die einschlägige Offenbarung der DE-A-10,2010,044204). Dabei resultiert eine Zusammensetzung, die die Verbindungen (I) sowie die resultierenden Abfangprodukte enthält.

### Verwendung der Zusammensetzungen

Ein weiterer Erfindungsgegenstand ist die Verwendung der Verbindungen (I) zur Beschichtung der Oberflächen fester Substrate. Dabei ist die Art des Substrats an sich nicht beschränkt. Beispiele für geeignete Substrate sind beispielsweise Textil, Leder, Metall, Kunststoff, Glas, Holz, Papier oder Pappe.

In einer besonders bevorzugten Ausführungsform handelt es sich bei den Substraten um Kunststoffe.

Als Kunststoffe werden - dem üblichen Sprachgebrauch folgend -im Rahmen der vorliegenden Anmeldung organische, polymere Festkörper bezeichnet. Typischerweise teilt man die Kunststoffe in drei großen Gruppen ein, nämlich in Thermoplaste, Duroplaste und Elastomere. Umgangssprachlich werden Kunststoffe auch als Plastik bezeichnet. Üblicherweise werden Kunststoffe synthetisch oder halbsynthetisch aus monomern organischen Molekülen oder Biopolymeren hergestellt.

Geeignete Substrate für die erfindungsgemäßen Beschichtungsmassen sind beispielsweise thermoplastische Polymere, insbesondere Polymethylmethacrylate, Polybutylmethacrylate, Polyethylenterephthalate, Polybutylenterephthalate, Polyvinylidenfluoride, Polyvinylchloride, Polyester, Polyolefine, Acrylnitrilethylenpropylendienstryolcopolymere (A-EPDM), Polyetherimide, Polyetherketone, Polyphenylensulfide, Polyphenylenether oder deren Mischungen.

Weiterhin genannt seien Polyethylen, Polypropylen, Polystyrol, Polybutadien, Poly-ester, Polyamide, Polyether, Polycarbonat, Polyvinylacetal, Polyacrylnitril, Polyacetal, Polyvinylalkohol, Polyvinylacetat, Phenolharze, Harnstoffharze, Melaminharze, Alkydharze, Epoxidharze oder Polyurethane, deren Block- oder Pfropfcopolymere und Blends davon.

Folgende Kunststoffe seien als bevorzugt geeignete Kunststoffe genannt: Acrylnitril-Butadien-Styrol (ABS), Polyacrylnitril/Methylmethacrylat (AMMA), Acrylnitril-Styrol-Acrylester (ASA), Epoxidharze (EP), expandiertes Polystyrol (EPS), Ethylen-Vinylacetat Copolymer (EVA), high densitiy Polyethylen (HDPE), low densitiy Polyethylen (LDPE), Methylmethacrylat/Acrylnitril/Butadien/Styrol (MABS), Methylacrylat / Butadien /Styrol Copolymer (MBS), Melamin-Formaldehyd-Harz (MF), Polyamid (PA), Nylon (PA6), Nylon (PA66), Polyacrylnitril (PAN), 1,2-Polybutadien (PB), Polybutylenterephthalat (PBT), Polycarbonat (PC), Polyethylen (PE), chloriertes Polyethylen (PEC), Polyetheretherketon (PEEK), Polyetherimid (PEI), Polyetherketon (PEK), Polyarylethersulfon (PES), Polyethylenterephthalat (PET), Phenol-Formaldehyd-Harz (PF), Polyimid (PI), Polyisobutylen (PIB), Polymethylmethacrylat (PMMA), Polyoxymethylen (POM), Polypropylen (PP), Polyethylensulfid (PPS), Polystyrol (PS), Polysulfon (PSU), Polyurethan (PUR), Polyvinylacetat (PVAC), Polyvinylalkohol (PVAL), Polyvinylchlorid (PVC), Polyvinylidenchlorid (PVDC), Styrol-Acrylnirtil (SAN), Styrol-Butadien (SB), Harnstoff-Formaldehyd-Harz (UF), Ungesättiges Polyesterharz UP-Kunststoffe (Kurzzeichen gemäß DIN 7728) und aliphatische Polyketone.

Besonders bevorzugte Substrate sind Polyolefine, wie z.B. PP (Polypropylen), das wahlweise isotaktisch, syndiotaktisch oder ataktisch und wahlweise nicht-orientiert oder durch uni- oder bisaxiales Recken orientiert sein kann, SAN (Styrol-Acrylnitril-Copolymere), PC (Polycarbonate), PVC (Polyvinylchloride), PMMA (Polymethylmethacrylate), PBT (Poly(butylenterephthalate), PA (Polyamide), ASA (Acrylnitril-Styrol-Acrylester-Copolymere) und ABS (Acrylnitril-Butadien-Styrol-Copolymere), sowie deren physikalische Mischungen (Blends). Besonders bevorzugt sind PP, SAN, ABS, ASA sowie Blends von ABS oder ASA mit PA oder PBT oder PC.

Ein weiterer Erfindungsgegenstand ist die Verwendung von Beschichtungsmassen, enthaltend ein oder mehrere Verbindungen (I) zur Beschichtung der Oberflächen fester Substrate. Dabei ist die Art des Substrats an sich nicht beschränkt. In einer besonders bevorzugten Ausführungsform handelt es sich bei den Substraten um Kunststoffe, für die das oben Gesagte gilt.

Der Begriff "Beschichtungsmassen" umfasst jegliche Art von Zusammensetzungen, die auf die Oberfläche eines zu beschichtenden Substrates aufgetragen und anschließend - gegebenenfalls nach vorheriger Trocknung - ausgehärtet wird. Insbesondere schließt der Begriff "Beschichtungsmassen" alle Lackarten ein.
Dabei ist - wie dem Fachmann bekannt - unter "Lack" eine Beschichtungsmasse zu verstehen, die flüssig oder auch pulverförmig sein kann, und die in dünner Schicht dünn auf einen Gegenstand, also das zu beschichtende Substrat, aufgetragen wird und dann ausgehärtet wird. Vergleiche hierzu auch den unten stehenden Abschnitt zum Begriff "Beschichten".

Die erfindungsgemäßen Beschichtungsmassen können neben den Verbindungen (I) zusätzlich weitere lacktypische Additive enthalten, beispielsweise Antioxidantien, Stabilisatoren, Aktivatoren (Beschleuniger), Füllmittel, Pigmente, Farbstoffe, antistatische Agentien, Flammschutzmittel, Verdicker, thixotrope Agentien, oberflächenaktive Agentien, Viskositätsmodifikatoren, Plastifizierer oder Chelatbildner verwendet werden. Weiterhin können die erfindungsgemäßen Beschichtungsmassen neben den Verbindungen (I) auch weitere strahlungshärtbare Komponenten enthalten, die nicht von der Formel (I) umfasst sind.

Als Verdicker kommen neben radikalisch (co)polymerisierten (Co)Polymerisaten, übliche organische und anorganische Verdicker wie Hydroxymethylcellulose oder Bentonit in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin® -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperi-dyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Pigmente können ebenfalls in den Beschichtungsmassen enthalten sein. Pigmente sind gemäß CD Römpp Chemie Lexikon - Version 1.0, Stuttgart/New York: Georg Thieme Verlag 1995 unter Verweis auf DIN 55943 partikelförmige "im Anwendungsmedium praktisch unlösliche, anorganische oder organische, bunte oder unbunte Farbmittel". Praktisch unlöslich bedeutet dabei eine Löslichkeit bei 25 °C unter 1 g / 1000 g Anwendungsmedium, bevorzugt unter 0,5, besonders bevorzugt unter 0,25, ganz besonders bevorzugt unter 0,1 und insbesondere unter 0,05 g / 1000 g Anwendungsmedium.

Wird ein Pigment eingesetzt, so ist darauf zu achten, dass entweder die Härtung mit Elektronenstrahlen durchgeführt wird oder dass ein Photoinitiator verwendet wird, der trotz der Pigmentierung durch die eingestrahlte Strahlung aktiviert werden kann, beispielsweise indem der Photoinitiator eine signifikante Absorbanz in einem Wellenlängenbereich aufweist, in dem das Pigment für die eingestrahlte Strahlung ausreichend durchlässig ist. Es stellt eine bevorzugte Ausführungsform der vorliegenden Erfindung dar, kein Pigment zu verwenden und die Beschichtungsmasse in Klarlacken einzusetzen.

Beispiele für Pigmente umfassen beliebige Systeme von Absorptions- und/oder Effektpigmenten, bevorzugt Absorptionspigmente. Anzahl und Auswahl der Pigmentkomponenten sind dabei keinerlei Beschränkungen unterworfen. Sie können den jeweiligen Erfordernissen, beispielsweise dem gewünschten Farbeindruck, beliebig angepaßt werden.

Unter Effektpigmenten sind alle Pigmente zu verstehen, die einen plättchenförmigen Aufbau zeigen und einer Oberflächenbeschichtung spezielle dekorative Farbeffekte verleihen. Bei den Effektpigmenten handelt es sich beispielsweise um alle in der Fahrzeug- und Industrielackierung üblicherweise einsetzbaren effektgebenden Pigmente. Beispiele für derartige Effektpigmente sind reine Metallpigmente; wie z.B. Aluminium-, Eisen- oder Kupferpigmente; Interferenzpigmente, wie z.B. titandioxidbeschichteter Glimmer, eisenoxidbeschichteter Glimmer, mischoxidbeschichteter Glimmer (z.B. mit Titandioxid und Fe₂O₃ oder Titandioxid und Cr₂O₃), metalloxidbeschichtetes Aluminium, oder Flüssigkristallpigmente.

Bei den farbgebenden Absorptionspigmenten handelt es sich beispielsweise um übliche in der Lackindustrie einsetzbare organische oder anorganische Absorptionspigmente. Beispiele für organische Absorptionspigmente sind Azopigmente, Phthalocyanin-, Chinacridon- und Pyrrolopyrrolpigmente. Beispiele für anorganische Absorptionspigmente sind Eisenoxidpigmente, Titandioxid und Ruß.

Sofern die Aushärtung der Beschichtungsmassen nicht mit Elektronenstrahlen, sondern mittels UV-Strahlung erfolgt, ist vorzugsweise wenigstens ein Photoinitiator enthalten, der die Polymerisation ethylenisch ungesättigter Doppelbindungen (C=C-Doppelbindungen) initiieren kann. Ganz allgemein können alle dem Fachmann einschlägig bekannten Photoinitiatoren ein gesetzt werden, wie sie beispielsweise in einschlägigen Fachpublikationen und Monographien beschrieben sind.

In Betracht kommen zum Beispiel:
- Mono- oder Bisacylphosphinoxide, etwa 2,4,6-Trimethylbenzoyldiphenylphosphinoxid (Lucirin® TPO der BASF SE), Ethyl-2,4,6-trimethylbenzoylphenylphosphinat (Lucirin® TPO L der BASF SE), Bis-(2,4,6-trimethylbenzoyl)-phenylphosphinoxid (Irgacure® 819 der Firma Ciba Spezialitätenchemie),
- Benzophenone, Hydroxyacetophenone, Phenylglyoxylsäure und ihre Derivate oder Gemische dieser Photoinitiatoren. Als Beispiele seien genannt: Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon, β-Methylanthrachinon, tert-Butylanthrachinon, Anthrachinoncarbonysäureester, Benzaldehyd, α-Tetralon, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon, Benzoin, Benzoin-iso-butylether, Chloroxanthenon, Benzointetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Tri-o-Tolylphosphin, Benz[a]anthracen-7,12-dion, 2,2-Diethoxyacetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureester-Typ.

Es können auch Gemische verschiedener Photoinitiatoren eingesetzt werden. Typische Gemische umfassen beispielsweise 2-Hydroxy-2-Methyl-1-phenyl-propan-2-on und 1-Hydroxycyclohexyl-phenylketon, Bis(2,6-dimethoxybenzoyl)-2,4,4-trimethylpentylphosphinoxid und 2-Hydroxy-2-methyl-1-phenyl-propan-1-on, Benzophenon und 1-Hydroxy-cyclohexylphenylketon, Bis(2,6-dimethoxybenzoyl)-2,4,4-trimethylpentylphosphinoxid und 1-Hydroxycyclohexyl-phenylketon, 2,4,6-Trimethylbenzoyldiphenylphosphinoxid und 2-Hydroxy-2-methyl-1-phenyl-propan-1-on, 2,4,6-Trimethylbenzophenon und 4-Methylbenzophenon oder 2,4,6-Trimethylbenzophenon und 4-Methylbenzophenon und 2,4,6-Trimethylbenzoyldiphenylphosphinoxid.

Bevorzugte Photoinitiatoren sind:
- 2,4,6-Trimethylbenzoyldiphenylphosphinoxid,
- Ethyl-2,4,6-trimethylbenzoylphenylphosphinat,
- Bis-(2,4,6-trimethylbenzoyl)-phenylphosphinoxid,
- Benzophenon,
- 1-Benzoylcyclohexan-1-ol,
- 2-Hydroxy-2,2-dimethylacetophenon und
- 2,2-Dimethoxy-2-phenylacetophenon.

Die Beschichtungsmassen enthalten die Photoinitiatoren vorzugsweise in einer Menge von 0,05 bis 10 Gew.-%, besonders bevorzugt 0,1 bis 8 Gew.-%, insbesondere 0,2 bis 5 Gew.-%, bezogen auf die Gesamtmenge der in den Beschichtungsmassen vorhandenen härtbaren Komponenten.

Die Beschichtung der Oberflächen fester Substrate mit den erfindungsgemäß einzusetzenden Tetrahydrofuranderivaten (I) erfolgt nach üblichen, dem Fachmann bekannten Verfahren, wobei man das gewünschte Tetrahydrofuranderivat (I) bzw. eine Beschichtungsmasse, die ein oder mehrere Verbindungen (I) enthält in der gewünschten Stärke auf das Substrat aufbringt und zumindest teilweise strahlungshärtet. Dabei ist eine vollständige Strahlenhärtung bevorzugt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Co-Extrudieren, bevorzugt durch Spritzen und Walzen erfolgen. Als Spritzverfahren können z.B. Luftdruck-, Airless- oder Elektrostatik-Spritzverfahren Anwendung finden.

Wie dem Fachmann bekannt ist unter Strahlenhärtung die radikalische Polymerisation von polymerisierbaren Verbindungen zu verstehen, die induziert wird durch elektromagnetische und/oder korpuskularen Strahlung. Der Einsatz von UV-Licht oder Elektronenstrahlung (Elektronenstrahlung; 150 bis 300 keV) ist bevorzugt. Insbesondere ist UV-Licht im Wellenlängenbereich von 200 bis 500 nm und insbesondere von 250 bis 400 nm bevorzugt.

Die Beschichtungsstärke wird vorzugsweise so eingestellt, dass die Trockenfilmdicke im Bereich von 30 bis 200 µm, und vorzugsweise im Bereich von 50-150 µm liegt. Wie dem Fachmann bekannt, versteht man unter Trockenschichtdicke die Schichtdicke einer getrockneten bzw. ausgehärteten Beschichtung. Der Begriff des Trocknens schließt dabei ein, dass in einer Beschichtungsmasse vorhandene Lösungsmittel, z.B. Wasser oder organische Lösungsmittel, verdunstet sind. Der Begriff des Aushärtens schließt dabei ein, dass eine Vernetzung der Beschichtungsmasse erfolgt. Es sei eigens betont, dass der Begriff der Trockenschichtdicke hier rein phänomenologisch zu verstehen ist als diejenige Schichtdicke, die eine getrocknete und/oder ausgehärteten Beschichtung aufweist.

Die Strahlenhärtung kann gewünschtenfalls bei höheren Temperaturen erfolgen. Bevorzugt ist dabei eine Temperatur oberhalb der T_{g}-Wertes des strahlungshärtbaren Bindemittels (T_{g}-Wert = Glasübergangstemperatur).

Die Strahlenhärtung kann unter sauerstoffhaltiger Atmosphäre oder unter Inertgas erfolgen, wobei letzteres bevorzugt ist.

Neben einer Strahlenhärtung können noch weitere Härtungsmechanismen involviert sein, beispielsweise thermische-, Feuchtigkeits-, chemische und/oder oxidative Härtung. Gegebenenfalls kann, wenn mehrere Schichten des Beschichtungsmittels übereinander aufgetragen werden, nach jedem Beschichtungsvorgang eine Trocknung und/oder Strahlenhärtung erfolgen.

Als Strahlungsquellen für die Strahlenhärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Laser, gepulste Lampen (Blitzlicht), Halogenlampen Elektronenblitzeinrichtungen, wodurch eine Strahlenhärtung ohne Photoinitiator möglich ist, oder Excimerstrahler.

Es können auch mehrere Strahlungsquellen für die Strahlenhärtung eingesetzt werden, z.B. zwei bis vier. Diese können gewünschtenfalls auch in jeweils unterschiedlichen Wellenlängebereichen strahlen.

Die Bestrahlung kann gegebenenfalls auch unter Ausschluss von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase.

Ein weiterer Erfindungsgegenstand ist gemäß dem oben Ausgeführten ein Verfahren zum Beschichten der Oberflächen fester Substrate, wobei man Tetrahydrofuranderivate (I) 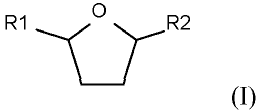 worin der Rest R1 die Bedeutung (CH₂=CH-CO-O-(CHR3-CH₂-O)ₘ-CH₂)- und der Rest R2 die Bedeutung (CH₂=CH-CO-O-(CHR4-CH₂-O)ₙ-CH₂)- hat, wobei die Reste R3 und R4 unabhängig voneinander Wasserstoff oder Methyl bedeuten, und wobei die Maßgabe gilt, dass die Summe der Indices m und n eine Zahl im Bereich von 5 bis 12 ist, oder Beschichtungsmassen, die ein oder mehrere Verbindungen (I) enthalten, auf die Oberfläche eines festen Substrates aufbringt und anschließend eine Strahlenhärtung durchführt, insbesondere mittels UV-Licht.

Unter "Beschichten", auch "coating" genannt, sind dabei Verfahren zu verstehen, die dem Aufbringen einer festhaftenden Schicht auf die Oberfläche eines Werkstückes - dem Substrat-dienen. Die aufgetragene Schicht wird als Beschichtung bezeichnet. Die üblichen Beschichtungsverfahren unterscheiden sich durch die Art der Aufbringung der Beschichtungsmassen in chemische, mechanische, thermische und thermomechanische Verfahren. Im Rahmen der vorliegenden Erfindung ist die UV-Härtung bevorzugt, die eine chemische Vernetzung der in den Beschichtungsmassen enthaltenen Verbindungen (I) induziert.

### Beispiele

### Mess- und Prüfmethoden

**Viskosität:** Die Viskosität der Substanzen als solche wurden gemessen mit einem Brookfield-Viskosimeter bei 25 °C, Geschwindigkeitsgefälle von 1000 s-1, gemäß DIN EN ISO 3219/A.3. **Pendeldämpfung (PD):** Die Pendeldämpfung (oft auch als Pendelhärte bezeichnet) von Beschichtungen, die resultierten, wenn die zu prüfenden Substanzen auf die Oberflächen fester Substrate aufgebracht und mittels UV-Strahlung gehärtet worden waren, die sogenannten Pendelhärte nach König, wurden gemessen nach DIN 53157. Bei dieser Methode wird die Pendeldämpfung in Sekunden angegeben.

**Erichsentiefung (Ew):** Die Erichsentiefung ist ein Maß für die Elastizität von Beschichtungen. Die Erichsentiefung von Beschichtungen, die resultierten, wenn die zu prüfenden Substanzen auf die Oberflächen fester Substrate aufgebracht und mittels UV-Strahlung gehärtet worden waren, wurden gemessen gemäß DIN ISO 1520.. Die Erichsentiefung wird in [mm] angegeben.

**Haftung (Gitterschnittwert = G-Wert):** Die Haftung auf Kunststoffen wurde bestimmt nach der Gitterschnitt-Methode gemäß DIN EN ISO 2409, wobei die G-Werte nach Schulnoten-System im Bereich von 0 bis 5 liegen. Dabei stellt 0 den besten und 5 den schlechtesten Wert der Skala dar. Als zu beschichtender Kunststoff wurde Stamylan eingesetzt.

**Iodfarbzahl:** Die Messung der Iodfarbzahl erfolgte mit dem Gerät Lange Lico 400 gemäß DIN 6162

### Eingesetzte Substanzen

**TBABr:** Tetrabutylammonium Bromid (CAS-Nr. 1643-19-2)

**Glycidether:** Pentaerythrit-di/tri-glycidether (CAS-Nr. 30973-88-7), "Ipox CL 16" (Fa. Ipox)

**THF-Diol:** 2,5-Dimethyloltetrahydrofuran; technische Mischung mit einem molaren cis/trans-Verhältnis von 90: 10.

**THF-Diol-5,3PO:** Umsetzungsprodukte von 7,5 mol THF-Diol mit 39,7 mol Propylenoxid. Die Herstellung erfolgte wie nachfolgend angegeben: 995.0 g THF-Diols und 10.0 g festes KOH wurden bei 25 °C in einen 5 L Reaktor vorgelegt. Anschließend wurde dieser mit Stickstoff inertisiert. Der Kessel wurde auf 120 °C erhitzt und 2300 g Propylenoxid zudosiert. Nach einer Abreaktionszeit von 4 h wurde für 30 Minuten unter vollem Vakuum bei 50 °C evakuiert und anschließend auf 25 °C abgekühlt. Die Aufarbeitung erfolgte durch Neutralisation mit Ionenaustauschmaterialien (Ambosol) und Wasser, Vakuumdestillation und Filtration. Das erhaltene Produkt war eine helle Flüssigkeit. Es wurden 3313,4 g Produkt erhalten. Der erhaltene Polyether besaß folgende Kennwerte:
OH-Zahl: 260 mg KOH/g
Viskosität (25 °C): 152 mPas

**THF-Diol-7EO:** Umsetzungsprodukte von 7.5 mol THF-Diol mit 52,3 mol Ethylenoxid. 990.0 g THF-Diol und 9.9 g festes KOH wurden bei 25 °C in einen 5 L Reaktor vorgelegt. Anschließend wurde dieser mit Stickstoff inertisiert. Der Kessel wurde auf 120 °C erhitzt und 2300 g Ethylenoxid zu dosiert. Nach einer Abreaktionszeit von 4 h wurde für 30 Minuten unter vollem Vakuum bei 50 °C evakuiert und anschließend auf 25 °C abgekühlt. Die Aufarbeitung erfolgte durch Neutralisation mit Ionenaustauschmaterialien (Ambosol) und Wasser, Vakuumdestillation und Filtration. Das erhaltene Produkt war eine helle Flüssigkeit. Es wurden 3240,3 g Produkt erhalten.

Der erhaltene Polyether besaß folgende Kennwerte:
OH-Zahl: 272 mg KOH/g
Viskosität (25 °C): 314 mPas

### Beispiele

### Beispiel 1 (Referenzbeispiel) Herstellung von THF-Diol-Diacrylat

Es wurden 115,35 g (1,75 mol OH) THF-Diol mit einer OH-Zahl von 424 mg KOH/g, 143,66 g Acrylsäure, 86,33 g Cyclohexan, und 10,4 g Methansulfonsäure 70% aqu. in Anwesenheit eines Stabilisatorgemisches aus 0,26g Kerobit, 0,78 g Methylhydrochinon, 0,27g Hypophosphorige Säure (50% aqu.) und 7,8 mg Phenothiazin, wobei das Stabilisatorgemisch in 1,0g Acrylsäure gelöst war, zusammengegeben und die Komponenten bei 90-95°C verestert. Nach 5 Stunden war ein Umsatz von 90% erreicht. Anschließend wurden das Cyclohexan und überschüssige Acrylsäure bis zu einer Säurezahl (SZ) von 49 mg KOH/g Substanz im Vakuum entfernt.
Viskosität: 27 mPas
Iodfarbzahl: 42

### Beispiel 2

### THF-Diol-7EO-Diacrylat

Es wurden in einem 500 ml Dreihalskolben 158,86 g (0,72 mol OH) THF-7EO, 59,41 g Acrylsäure, 86,33 g Methylcyclohexan, und 6,5 g Schwefelsäure conc in Anwesenheit eines Stabilisatorgemisches aus 1,0g Kerobit, 4,36g Methylhydrochinon und 4,36g Hypophosphoriger Säure (50% aqu.) zusammengegeben und die Komponenten bei 101-105 °C verestert. Innerhalb von 6,5 Stunden wurde ein Umsatz von 78% erreicht. Anschließend wurden das Methylcyclohexan und das überschüssige Acrylsäure bis zu einer Säurezahl (SZ) von 53 mg KOH/g Substanz im Vakuum entfernt. Dann erfolgte eine Umsetzung der Restacrylsäure in 200g Rohester mit 31,7g Ipox CL 16 unter TBABr-Katalyse (4g) bei 107-108 °C bis eine Säurezahl (SZ) von 4,0 erreicht wurde und anschließender Filtration des Produkts über einen Seitz K300 Filter. Das Produkt - eine Mischung von THF-Diol-7EO-Diacrylat und den Abfangprodukten (= Produkten der Umsetzung überschüssigen Acrylsäure mit Ipox CL 16) wurde wie folgt charakterisiert:
Viskosität: 340 mPas
Iodfarbzahl: 0,9

### Beispiel 3

### Herstellung von THF-Diol-5,3 PO-Diacrylat

Es wurden in einem 2000 ml Dreihalskolben 884,76 g (4,0 mol OH) THF-Diol-5,3 PO, 315,23 g Acrylsäure, 400 g Methylcyclohexan, und 8,1 g Methansulfonsäure (70%aqu.) in Anwesenheit eines Stabilisatorgemisches aus 6,0g Kerobit, 24,0g Methylhydrochinon und 24,0g Hypophosphoriger Säure (50% aqu.) zusammengegeben und die Komponenten bei 101-105 C° verestert. Innerhalb von 8 Stunden wurde ein Umsatz von 79% erreicht. Anschließend wurden das Methylcyclohexan und überschüssige Acrylsäure bis zu einer Säurezahl (SZ) von 46 mg KOH/g Substanz im Vakuum entfernt. Dann erfolgte eine Umsetzung der Restacryläure mit 155,3g Ipox CL 16 unter TBABr-Katalyse (25,71g) bei 107-108 °C bis eine SZ von 3,9 erreicht wurde und anschließender Filtration des Produkts über einen Seitz K300 Filter. Das Produkt - eine Mischung von THF-Diol-7PO-Diacrylat und den Abfangprodukten (= Produkten der Umsetzung überschüssigen Acrylsäure mit Ipox CL 16) wurde wie folgt charakterisiert:
Viskosität: 460 mPas
Iodfarbzahl: 0,4

### Beispiel 4

### Herstellung von THF-Diol-5,3PO-Diacrylat

Es wurden in einem 2000 ml Dreihalskolben 879,51 g (4,0 mol OH) THF-Diol-5,3 PO, 320,49 g Acrylsäure, 228 g Cyclohexan, und 75,30g p-Toluolsulfonsäure (65% aqu.) in Anwesenheit eines Stabilisatorgemisches aus 0,86g einer 31,5 Gew.%-igen wässrigen Lösung von CuCl₂, 0,24g Methylhydrochinon und 3g H₃PO₂ zusammengegeben und die Komponenten bei 99 C° verestert. Innerhalb von 8 Stunden wurde ein Umsatz von 79% erreicht. Die Aufarbeitung erfolgt durch wässrige Extraktion der überschüssigen Acrylsäure, Entfernen des Lösungsmittels bei reduziertem Druck und anschließender Filtration des Produkts über einen Seitz K300 Filter. Das Produkt wurde wie folgt charakterisiert:
Viskosität: 70 mPas
Iodfarbzahl: 5,2

### Anwendungsbeispiele

In den folgenden Anwendungsbeispielen wurde zur Aushärtung der Beschichtungsmassen eine IST-UV-Anlage eingesetzt (Anlagentyp: M-40-2x1-R-TR-SLC-SO-Inert; Lampe 1: IST-UV-Lampe M400 U2HC; Lampe 2: IST-UV-Lampe M400 U2H)

### Anwendungsbeispiel 1

### (Referenzbeispiel)

### Bestimmung der Filmeigenschaften des Produktes gemäß Beispiel 1

Zu dem Produkt gemäß Beispiel 1 wurden 5 Gew % - bezogen auf dieses Produkt - des Photoinitiators Irgacure 500 gegeben. Die so hergestellte Beschichtungsmasse wurde mit einem Kastenrakel auf Stamylan aufgebracht, wobei die Spaltbreite des Rakels 200 µm betrug(damit ist impliziert, dass die Nassfilmdicke der aufgebrachten Beschichtung 200 µm betrug). Die Aushärtung erfolgte unter Stickstoffatmosphäre mit einem Energieeintrag von 1900 mJ/cm². Anschließend erfolgte die Bestimmung von Pendeldämpfung (PD), Erichsentiefung (Ew) und Gitterschnitt-Wert. Dabei wurden folgende Ergebnisse erhalten:
PD = 113 s
Ew = 1,6 mm
Gitterschnitt-Wert = 0

### Anwendungsbeispiel 2

### Bestimmung der Filmeigenschaften des Produktes gemäß Beispiel 2

Zu dem Produkt gemäß Beispiel 2 wurden 5 Gew % - bezogen auf dieses Produkt - des Photoinitiators Irgacure 500 gegeben. Die so hergestellte Beschichtungsmasse wurde mit einem Kastenrakel auf Stamylan aufgebracht, wobei die Spaltbreite des Rakels 200 µm betrug(damit ist impliziert, dass die Nassfilmdicke der aufgebrachten Beschichtung 200 µm betrug). Die Aushärtung erfolgte unter Stickstoffatmosphäre mit einem Energieeintrag von 1900 mJ/cm2. Anschließend erfolgte die Bestimmung von Pendeldämpfung (PD), Erichsentiefung (Ew) und Gitterschnitt -Wert. Dabei wurden folgende Ergebnisse erhalten:
PD = 88 s
Ew = 4,1 mm
Gitterschnitt -Wert = 1

### Anwendungsbeispiel 3

### Bestimmung der Filmeigenschaften des Produktes gemäß Beispiel 3

Zu dem Produkt gemäß Beispiel 3 wurden 5 Gew % - bezogen auf dieses Produkt - des Photoinitiators Irgacure 500 gegeben. Die so hergestellte Beschichtungsmasse wurde mit einem Kastenrakel auf Stamylan aufgebracht, wobei die Spaltbreite des Rakels 100 µm betrug(damit ist impliziert, dass die Nassfilmdicke der aufgebrachten Beschichtung 100 µm betrug). Die Aushärtung erfolgte unter Stickstoffatmosphäre mit einem Energieeintrag von 1900 mJ/cm². Anschließend erfolgte die Bestimmung von Pendeldämpfung (PD), Erichsentiefung (Ew) und Gitterschnitt -Wert. Dabei wurden folgende Ergebnisse erhalten:
PD = 123 s
Ew = 3,3 mm
Gitterschnitt -Wert = 1

### Anwendungsbeispiel 4

### Bestimmung der Filmeigenschaften des Produktes gemäß Beispiel 4

Zu dem Produkt gemäß Beispiel 4 wurden 5 Gew % - bezogen auf dieses Produkt - des Photoinitiators Irgacure 500 gegeben. Die so hergestellte Beschichtungsmasse wurde mit einem Kastenrakel auf Stamylan aufgebracht, wobei die Spaltbreite des Rakels 100 µm betrug(damit ist impliziert, dass die Nassfilmdicke der aufgebrachten Beschichtung 100 µm betrug). Die Aushärtung erfolgte unter Stickstoffatmosphäre mit einem Energieeintrag von 1900 mJ/cm2. Zugabe von 5% Irgacure 500 als Photoinitiator; Auftrag mit Kastenrakel auf Stamylan; Spaltbreite Rakel 100 µm (damit ist impliziert, dass die Nassfilmdicke der aufgebrachten Beschichtung 100 µm beträgt); Härtung unter Stickstoffatmosphäre mit einem Energieeintrag von 1900 mJ/cm². Anschließend erfolgte die Bestimmung von Pendeldämpfung (PD), Erichsentiefung (Ew) und Gitterschnitt -Wert. Dabei wurden folgende Ergebnisse erhalten:
PD = 141 s
Ew = 3,1 mm
Gitterschnitt -Wert = 0

## Patentansprüche

1. Tetrahydrofuran-Derivate der Formel (I) 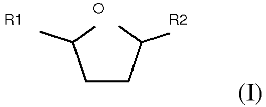 worin der Rest R1 die Bedeutung (CH₂=CH-CO-O-(CHR3-CH₂-O)ₘ-CH₂)- und der Rest R2 die Bedeutung (CH₂=CH-CO-O-(CHR4-CH₂-O)ₙ-CH₂)- hat, wobei die Reste R3 und R4 unabhängig voneinander Wasserstoff oder Methyl bedeuten, und wobei die Maßgabe gilt, dass die Summe der Indices m und n eine Zahl im Bereich von 5 bis 12 ist.

2. Beschichtungsmassen enthaltend ein oder mehrere Tetrahydrofuran-Derivate der Formel (I) gemäß Anspruch 1.

3. Verwendung von Tetrahydrofuran-Derivaten der Formel (I) gemäß Anspruch 1 zur Beschichtung der Oberflächen fester Substrate.

4. Verwendung nach Anspruch 3, wobei es sich bei den festen Substraten um Kunststoffe handelt.

5. Verwendung von Beschichtungsmassen enthaltend ein oder mehrere Tetrahydrofuran-Derivate der Formel (I) gemäß Anspruch 1 zur Beschichtung der Oberflächen fester Substrate.

6. Verwendung nach Anspruch 5, wobei es sich bei den festen Substraten um Kunststoffe handelt.

7. Verfahren zum Beschichten der Oberflächen fester Substrate, wobei man Tetrahydrofuranderivate (I) gemäß Anspruch 1 oder Beschichtungsmassen, die ein oder mehrere Verbindungen (I) gemäß Anspruch 1 enthalten, auf die Oberfläche eines festen Substrates aufbringt und anschließend eine Strahlenhärtung durchführt.

8. Verfahren nach Anspruch 7, wobei es sich bei den festen Substraten um Kunststoffe handelt.

9. Verfahren nach Anspruch 7, wobei es sich bei der Strahlenhärtung um eine Härtung mit UV-Licht der Wellenlänge im Bereich von 200 bis 500 nm handelt.

10. Verfahren nach Anspruch 7, wobei es sich bei den festen Substraten um Kunststoffe und bei der Strahlenhärtung um eine Härtung mit UV-Licht der Wellenlänge im Bereich von 250 bis 400nm handelt.

## Claims

1. Tetrahydrofuran derivatives of the formula (I) 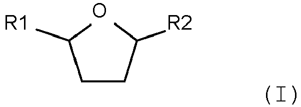 in which the radical R1 has the definition (CH₂=CH-CO-O-(CHR3-CH₂-O)ₘ-CH₂)- and the radical R2 has the definition (CH₂=CH-CO-O-(CHR4-CH₂-O)ₙ-CH₂)-, in which the radicals R3 and R4 independently of one another are hydrogen or methyl, with the proviso that the sum of the indices m and n is a number in the range from 5 to 12.

2. A coating composition comprising one or more tetrahydrofuran derivatives of the formula (I) according to claim 1.

3. The use of tetrahydrofuran derivatives of the formula (I) according to claim 1 for coating the surfaces of solid substrates.

4. The use according to claim 3, wherein the solid substrates are plastics.

5. The use of coating compositions comprising one or more tetrahydrofuran derivatives of the formula (I) according to claim 1 for coating the surfaces of solid substrates.

6. The use according to claim 5, wherein the solid substrates are plastics.

7. A method for coating the surfaces of solid substrates, by applying tetrahydrofuran derivatives (I) according to claim 1, or coating compositions which comprise one or more compounds (I) according to claim 1, to the surface of a solid substrate and carrying out radiation curing.

8. The method according to claim 7, wherein the solid substrates are plastics.

9. The method according to claim 7, wherein the radiation curing is curing with UV light of a wavelength in the range from 200 to 500 nm.

10. The method according to claim 7, wherein the solid substrates are plastics and the radiation curing is curing with UV light of a wavelength in the range from 250 to 400 nm.

## Revendications

1. Dérivés de tétrahydrofurane de formule (I) 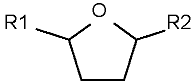 dans laquelle le radical R1 a la signification (CH₂=CH-CO-O-(CHR3-CH₂-O)ₘ-CH₂)- et le radical R2 a la signification (CH₂=CH-CO-O-(CHR4-CH₂-O)ₙ-CH₂)-, les radicaux R3 et R4 signifiant indépendamment l'un de l'autre hydrogène ou méthyle, à condition que la somme des indice m et n soit un nombre dans la plage allant de 5 à 12.

2. Matériaux de revêtement contenant un ou plusieurs dérivés de tétrahydrofurane de formule (I) selon la revendication 1.

3. Utilisation de dérivés de tétrahydrofurane de formule (I) selon la revendication 1 pour le revêtement de surfaces de substrats solides.

4. Utilisation selon la revendication 3, dans laquelle les substrats solides sont des plastiques.

5. Utilisation de matériaux de revêtement contenant un ou plusieurs dérivés de tétrahydrofurane de formule (I) selon la revendication 1 pour le revêtement de surfaces de substrats solides.

6. Utilisation selon la revendication 5, dans laquelle les substrats solides sont des plastiques.

7. Procédé de revêtement de surfaces de substrats solides, selon lequel des dérivés de tétrahydrofurane (I) selon la revendication 1 ou des matériaux de revêtement qui contiennent un ou plusieurs composés (I) selon la revendication 1 sont appliqués sur la surface d'un substrat solide, puis un durcissement par rayonnement est réalisé.

8. Procédé selon la revendication 7, dans lequel les substrats solides sont des plastiques.

9. Procédé selon la revendication 7, dans lequel le durcissement par rayonnement est un durcissement avec une lumière UV d'une longueur d'onde dans la plage allant de 200 à 500 nm.

10. Procédé selon la revendication 7, dans lequel les substrats solides sont des plastiques et le durcissement par rayonnement est un durcissement avec une lumière UV d'une longueur d'onde dans la plage allant de 250 à 400 nm.
